# EUROPÄISCHE PATENTSCHRIFT

(11) **EP 3 873 390 B1**
(45) Veröffentlichungstag und Bekanntmachung des Hinweises auf die Patenterteilung: **03.08.2022**
(21) Anmeldenummer: 19794508.2
(22) Anmeldetag: 23.10.2019
(51) Int. Cl.: A61F 5/01

(54) **KNIEORTHESE**
KNEE BRACE
ORTHÈSE DE GENOU

(30) Priorität: 29.10.2018 DE 102018126989
(43) Veröffentlichungstag der Anmeldung: 08.09.2021
(73) Patentinhaber: Ottobock SE & Co. KGaA, 37115 Duderstadt (DE)
(72) Erfinder: DREWITZ, Heiko, 37130 Gleichen (DE); TÜTTEMANN, Markus, 45731 Waltrop (DE); LIDOLT, Klaus, 37115 Duderstadt (DE); PUSCH, Martin, 37115 Duderstadt (DE); KRUCHEM, Tim, 37073 Göttingen (DE); MÜLLER, André, 37115 Duderstadt (DE); OVERDEVEST, Erienne, 37073 Göttingen (DE); KROLL-ORYWAHL, Olaf, 37154 Northeim (DE); SCHMITT, Alexander, 34123 Kassel (DE); SIEWERT, Gordon, 37083 Göttingen (DE)
(74) Vertreter: Gramm, Lins & Partner Patent- und Rechtsanwälte PartGmbB
(86) Internationale Anmeldenummer: PCT/EP2019/078942
(87) Internationale Veröffentlichungsnummer: WO 2020/089017

(56) Entgegenhaltungen:
- DE-A1- 10 143 067
- DE-A1- 19 811 925

## Beschreibung

Die Erfindung betrifft eine Knieorthese mit einem proximalen Rahmen und einem distalen Rahmen, die durch ein Lateralgelenk und/oder ein Medialgelenk schwenkbar aneinander angeordnet sind, wobei die Knieorthese ein proximales Druckelement, das mittels eines proximalen Stützarmes ausschließlich lateral oder aus-schließlich medial an dem proximalen Rahmen angeordnet ist, und ein distales Druckelement aufweist, das mittels eines distalen Stützarmes aus-schließlich lateral oder ausschließlich medial an dem distalen Rahmen angeordnet ist, wie in den Ansprüchen definiert.

Knieorthesen sind in unterschiedlichsten Ausführungsformen aus dem Stand der Technik bekannt und werden beispielsweise verwendet, um eine Osteoarthritis im Knie zu behandeln. Sie sind als einseitige Knieorthesen oder als zweiseitige Knieorthesen bekannt.

Aus der WO 2004/032793 A1 ist eine einseitige Knieorthese bekannt. Sie verfügt über einen proximalen Anteil, der am Oberschenkel des Trägers angeordnet wird, und einen distalen Anteil, der am Unterschenkel des Trägers angeordnet wird. Bei-de sind durch ein Gelenk miteinander verbunden, wodurch die Kniebewegung ermöglicht wird. Derartige Orthesen werden in vielen Fällen dazu verwendet, um eine Kraft auf das Knie aufzubringen, um die Schmerzen einer Osteoarthritis zu lindern. Dabei wird in der Regel vom Dreipunktprinzip ausgegangen. Bei diesem Prinzip liegt die Orthese an drei Punkten am Bein des Trägers an, an denen die Kraft aufgebracht wird. Der mittlere der drei Punkte befindet sich am Knie des Trägers und wird häufig durch eine Kniepelotte, die auf der dem Knie zugewandten Seite des jeweiligen Gelenkes angeordnet ist, realisiert. Am Oberschenkel und am Unter-schenkel liegen die beiden anderen Punkte. Dabei ist wichtig, dass der mittlere Kraftaufbringpunkt auf einer Knieseite, also medial oder lateral, liegt, während die beiden anderen Aufbringpunkte auf der gegenüberliegenden Seite liegen. Eine einseitige Knieorthese ist konstruktiv relativ einfach herzustellen, da erstmal eine Verbindung zwischen dem proximalen Anteil der Orthese und dem distalen Anteil der Orthese gibt. Dieser wird durch das eine Gelenk realisiert. Nachteilig ist jedoch, dass eine derartige einseitige Knieorthese nur eine relativ geringe Stabilität aufweist. Eine andere Form eines einseitigen Knieorthese ist aus der DE 101 43 067 A1 bekannt. Darin werden aufblasbare Kissen verwendet, um den von der Orthese aufzubringenden Druck einzustellen.

Die DE 198 11 925 A1 beschreibt eine Knieorthese, bei der die aufzubringenden Kräfte nicht in medialer und lateraler Richtung, sondern ventral und dorals aufzubringen sind.

Weiterhin sind aus dem Stand der Technik, beispielsweise der DE 60035431 T2, auch zweiseitige Knieorthesen bekannt. Sie verfügen über einen proximalen Rahmen und einen distalen Rahmen, die über zwei Gelenke, nämlich ein Lateralgelenk und ein Medialgelenk, schwenkbar aneinander angeordnet sind. Das Lateralgelenk befindet sich im angelegten Zustand der Knieorthese lateral des Knies, während das Medialgelenk medial des Knies angeordnet wird. Durch diese Ausgestaltung wird eine erhöhte Stabilität erreicht. Da jedoch die durch die oberen und unteren Kraftaufbringpunkte aufzubringende Kraft einstellbar ausgebildet sein sollte, müssen Verstelleinrichtungen in dem proximalen und/oder dem distalen Rahmen vorhanden sein, die eine Einstellung der in lateraler oder medialer Richtung aufzubringenden Kraft ermöglichen. Gleichzeitig dürfen diese Einstellmechanismen möglichst keine zusätzlichen oder zumindest keine übermäßigen Belastungen auf das Medialgelenk und/oder das Lateralgelenk aufbringen und dennoch eine Kniebewegung erlauben. Derartige Orthesen sind daher konstruktiv aufwendig und daher auch kostenintensiv.

Der Erfindung liegt die Aufgabe zugrunde, eine Knieorthese so weiterzuentwickeln, dass sie konstruktiv einfach herstellbar ist und dennoch eine Einstellbarkeit der aufzubringenden Kräfte erlaubt.

Die Erfindung löst die gestellte Aufgabe durch eine Knieorthese gemäß dem Oberbegriff des Anspruchs 1, die sich dadurch auszeichnet, dass die Knieorthese ein Koppelelement ausweist, das mit einem ersten Ende an dem proximalen Stützarm und mit einem zweiten Ende an dem distalen Rahmen oder mit dem ersten Ende an dem distalen Stützarm und mit dem zweiten Ende an dem proximalen Rahmen angeordnet ist.

Dies bedeutet, dass weder der proximale Rahmen noch der distale Rahmen eine Verschwenkbarkeit in der Frontalebene ermöglichen müssen. Dies wird bei zweiseitigen Knieorthesen im Stand der Technik benötigt, um die durch das proximale und das distale Druckelement aufzubringenden Kräfte einstellbar ausgestalten zu können. Da der proximale Rahmen und der distale Rahmen bei einer erfindungsgemäßen Knieorthese diese Einstellbarkeit nicht aufweisen müssen, können sie konstruktiv einfach ausgestaltet sein. Insbesondere werden auch keine innerhalb der Frontalebene wirkende zusätzlichen Kräfte auf das Medialgelenk und das Lateralgelenk ausgeübt, so dass auch diese als einfache, konstruktiv mit wenigen Bauteilen zu konstruierende Gelenke ausgebildet sein können, wie sie aus dem Stand der Technik bekannt sind.

Die für die Dreipunktwirkung der zweiseitigen erfindungsgemäßen Knieorthese nötigen Kräfte in Lateral- oder Medialrichtung werden durch das proximale Druckelement, das im angelegten Zustand der Knieorthese am Oberschenkel des Trägers angeordnet wird, und das distale Druckelement, das im angelegten Zustand der Knieorthese am Unterschenkel des Trägers angeordnet ist, hervorgerufen. Beide Druckelemente sind über jeweils einen Stützarm an einem der beiden Rahmen befestigt. Diese Befestigung erfolgt jedoch nicht zweiseitig, sondern ausschließlich lateral oder ausschließlich medial. Die Befestigung der Stützarme auf der Lateralseite der Knieorthese hat den Vorteil, dass die Verbindung leicht zugänglich ist und zwischen den Beinen des Trägers kein zusätzlicher Bauraum benötigt wird.

Vorzugsweise sind der proximale Stützarm und/oder der distale Stützarm lateral am jeweiligen Rahmen angeordnet, wenn die Knieorthese kein Medialgelenk, sondern lediglich ein Lateralgelenk, aufweist. Wenn die Knieorthese kein Lateralgelenk, sondern lediglich ein Medialgelenk aufweist sind der proximale Stützarm und/oder der distale Stützarm vorzugsweise medial am jeweiligen Rahmen angeordnet.

Selbstverständlich ist dies jedoch auf andersherum möglich. Gegebenenfalls ist einer der Stützarme lateral und der andere Stützarm medial angeordnet. Dies kann sowohl bei einem Medialgelenk als auch bei einem Lateralgelenk der Fall sein.

Vorzugsweise verfügt die Knieorthese sowohl über ein Lateralgelenk als auch über ein Medialgelenk.

Bevorzugt erstreckt sich der proximale Rahmen und/oder der distale Rahmen von der medialen Seite bis zu der lateralen Seite der Knieorthese. Dies bedeutet vorzugsweise, dass im angelegten Zustand der Knieorthese ein Teil des jeweiligen Rahmens auf der lateralen Seite des Beines und ein andere Teil auf der medialen Seite des Beines angeordnet ist.

Vorzugsweise sind der proximale Stützarm und der distale Stützarm auf der gleichen Seite angeordnet. Sie sind folglich beide lateral oder beide medial an dem jeweiligen Rahmen angeordnet.

Vorteilhafterweise ist ein Winkel, in dem der proximale Stützarm und/oder der distale Stützarm an dem jeweiligen Rahmen angeordnet ist, über jeweils eine Einstelleinrichtung einstellbar. Eine Verstellung des Winkels führt bei gleichbleibender Länge des Stützarmes dazu, dass das jeweilige Druckelement nach lateral oder medial verschoben wird. Das kann daher auf die individuelle Beinform des Trägers der Knieorthese angepasst werden. Zudem ist es auf diese Weise möglich, die durch das Druckelement aufgebrachte Kraft einzustellen.

Alternativ oder zusätzlich zu der Einstellbarkeit des Winkels über die jeweils eine Einstelleinrichtung ist vorzugsweise die Länge des Stützarmes einstellbar. Auch dadurch kann das Druckelement nach lateral oder medial verschoben werden und so die durch das Druckelement auf das Bein des Trägers ausgeübte Kraft eingestellt werden.

Vorteilhafterweise ist der Winkel für den proximalen Stützarm und für den distalen Stützarm unabhängig voneinander einstellbar. Auf diese Weise ist es besonders einfach möglich, der individuellen Beinform des Trägers zu folgen. So ist es durchaus möglich, dass ein proximales Druckelement weiter nach lateral verschoben werden muss, als das distale Stützelement, um die gleiche Kraft auf den Unterschenkel und den Oberschenkel auszuüben.

Vorteilhafterweise ist die jeweilige Einstelleinrichtung arretierbar. Vorteilhafterweise ist sie stufenlos arretierbar. Damit lässt sich der Winkel, den der Stützarm relativ zum jeweiligen Rahmen, also der proximale Stützarm zum proximalen Rahmen und der distale Stützarm zum distalen Rahmen, aufweist, stufenlos einstellen und die Einstelleinrichtung kann in jeder dieser Positionen arretiert werden. Dies kann beispielsweise über eine Klemmvorrichtung, eine Schraubvorrichtung oder eine andere aus dem Stand der Technik prinzipiell bekannte Einstelleinrichtung erreicht werden.

In einer konstruktiv besonders einfachen Ausgestaltung erlauben das Lateralgelenk und das Medialgelenk eine Schwenkbewegung des proximalen Rahmens relativ zu dem distalen Rahmen ausschließlich in einer Ebene, die im angelegten Zustand der Knieorthese im Wesentlichen einer Sagittalebene entspricht. Anders ausgedrückt erlauben das Medialgelenk und das Lateralgelenk ausschließlich eine Flexion und Extension des Knies, jedoch keine Bewegung senkrecht zu dieser Ebene.

Vorteilhafterweise ist der proximale Stützarm und/oder der distale Stützarm lösbar an dem jeweiligen Rahmen angeordnet. Auf diese Weise ist er leicht auswechselbar, um beispielsweise geänderten körperlichen Anforderungen Rechnung zu tragen, den Stützarm zu reparieren oder auszutauschen oder zu reinigen.

Vorzugsweise sind das proximale Druckelement und das distale Druckelement der-art angeordnet, dass sie im angelegten Zustand der Knieorthese beide medial oder beide lateral am Bein des Trägers anliegen. Es befindet sich vorteilhafterweise ein drittes Druckelement am Lateralgelenk oder am Medialgelenk, wobei dieses dritte Druckelement auf der dem proximalen Druckelement und dem distalen Druckelement entgegengesetzten Seite des Knies angeordnet ist. Auf diese Weise kann die prinzipiell bekannte Dreipunktwirkung erreicht werden. Das mittlere Druckelement kann, wie aus dem Stand der Technik bekannt, durch eine Kniepelotte ausgebildet sein, die am Medialgelenk, also dem Gelenk, das sich medial des Knies befindet, oder am Lateralgelenk, also dem Gelenk, das sich lateral des Knies befindet, angeordnet sein.

In einer bevorzugten Ausgestaltung sind die Druckelemente schwenkbar am jeweiligen Stützarm angeordnet. Dies ist insbesondere dann von Vorteil, wenn der Stützarm winkelverstellbar am jeweiligen Rahmen angeordnet ist. Auf diese Weise kann verhindert werden, dass sich auch ein Anlagewinkel des jeweiligen Stützelementes am Bein verändert.

Vorzugsweise sind die Druckelemente lösbar an den Stützarmen angeordnet. Auf diese Weise kann das Druckelement entfernt werden, um es beispielsweise zu reinigen, zu reparieren oder durch ein anderes, individuell passenderes Stützelement auszutauschen.

Erfindungsgemäß verfügt die Knieorthese über ein Koppelelement, das mit einem ersten Ende an dem proximalen Stützarm und mit einem zweiten Ende an dem distalen Rahmen oder mit dem ersten Ende an dem distalen Stützarm und mit dem zweiten Ende an dem proximalen Rahmen angeordnet ist. Dabei befinden sich die Befestigungsstellen der beiden Arme des Koppelelementes beide auf der medialen Seite oder beide auf der lateralen Seite der Orthese. Vorteilhafterweise sind die En-den des Koppelelementes medial an dem jeweiligen Stützarm und Rahmen angeordnet, wenn der Stützarm lateral am Rahmen angeordnet ist und umgekehrt. Ist der Stützarm folglich medial am jeweiligen Rahmen angeordnet, befinden sich die Befestigungsstellen für die beiden Enden des Koppelelementes auf der lateralen Seite. Selbstverständlich ist es jedoch auch möglich, dass die Befestigungsstellen der Enden des Koppelelementes und die Befestigung des jeweiligen Stützarmes an seinem Rahmenelement auf der gleichen Seite erfolgen.

Bei dem Koppelelement handelt es sich vorteilhafterweise um ein Element, das Druckkräfte übertragen kann. Dies kann beispielsweise eine entsprechende Feder oder ein starres Element, beispielsweise eine Stange sein.

Vorteilhafterweise ist das Koppelelement derart angeordnet, dass eine im angelegten Zustand der Knieorthese von dem proximalen Druckelement auf den Oberschenkel oder von dem distalen Druckelement auf den Unterschenkel aufgebrachte Kraft durch das Koppelelement beim Beugen des Knies variiert wird. Dabei kommt es auf die Kraft des Druckelementes an, das sich an dem Stützarm befindet, der mit dem Koppelelement verbunden ist.

Ist das Koppelelement beispielsweise mit dem ersten Ende an dem proximalen Stützarm angeordnet, ist das zweite Ende notwendigerweise am distalen Rahmen positioniert. Wird nun das Knie gebeugt, wird durch das Koppelelement und seine Druckkraft übertragende Wirkung die von dem proximalen Druckelement auf den Oberschenkel aufgebrachte Kraft variiert, wenn das Knie gebeugt wird. Wird das Koppelelement jedoch mit dem distalen Stützarm und dem proximalen Rahmen gekoppelt, verändert sich beim Beugen des Knies die von dem distalen Druckelement auf den Unterschenkel aufgebrachte Kraft.

Vorzugsweise wird diese Kraft bei einem Schwenkwinkel maximal, der größer ist als 10°, bevorzugt größer als 15°, besonders bevorzugt größer als 17° und kleiner als 30°, bevorzugt kleiner als 25°, besonders bevorzugt kleiner als 22° ist. Als besonders vorteilhaft hat sich herausgestellt, wenn dieser Schwenkwinkel, bei dem die aufgebrachte Kraft maximal wird, 22° Kniebeugung beträgt.

Vorzugsweise ist eine Länge des Koppelelementes veränderbar und feststellbar. Dies kann beispielsweise über Schraubelemente, Teleskopstangen oder ähnliche Elemente erreicht werden. Die Länge des Koppelelementes lässt sich einstellen, nachdem beispielsweise ein Verriegelungselement oder ein Befestigungselement gelöst wurde. Dies kann ein Klemmelement, eine Schraubverbindung oder ein anderes Formschlusselement sein. In diesem Zustand kann die Länge des Koppelelementes, beispielsweise in Form einer Teleskopstange, geändert werden. Ist die gewünschte Länge eingestellt, kann das Befestigungs- oder Verriegelungselement wieder befestigt werden und die Länge ist festgestellt.

Auch die geometrische Form des Koppelelementes kann gegebenenfalls einstellbar sein. So können Stangen gebogen, gerade oder gekrümmt ausgebildet sein.

Vorzugsweise befindet sich das proximale Druckelement auf der Seite, auf der der proximale Stützarm am proximalen Rahmen angeordnet ist. Alternativ oder zusätzlich dazu befindet sich bevorzugt das distale Druckelement auf der Seite, auf der der distale Stützarm am proximalen Rahmen angeordnet ist. Ist also der jeweilige Stützarm medial am jeweiligen Rahmen angeordnet, ist auch das betreffende Druckelement medial positioniert. Der Stützarm erstreckt sich folglich nicht von medial nach lateral, sondern erstreckt sich lediglich medial. Ist hingegen der jeweilige Stützarm lateral am jeweiligen Rahmen angeordnet, ist auch das betreffende Druckelement lateral positioniert. Der Stützarm erstreckt sich folglich nicht von lateral nach medial, sondern erstreckt sich lediglich lateral. Selbstverständlich sind auch die übrigen Möglichkeiten denkbar.

Mit Hilfe der beiliegenden Figuren wird nachfolgend ein Ausführungsbeispiel der vorliegenden Erfindung erläutert. Es zeigen:
- Figuren 1 und 2: - schematische dreidimensionale Ansichten einer Orthese gemäß einem ersten Ausführungsbeispiel der vorliegenden Er-findung,
- Figuren 3 und 4: - zwei Frontalansichten der in den Figuren 1 und 2 dargestellten Orthese,
- Figur 5: - eine Seitenansicht der gezeigten Orthese,
- Figur 6: - eine Orthese gemäß einem weiteren Ausführungsbeispiel der vorliegenden Erfindung während der Flexion,
- Figur 7: - eine Orthese gemäß einem weiteren Ausführungsbeispiel der vorliegenden Erfindung während der Extension,

- Figur 8: - die schematische Ansicht einer einseitigen Knieorthese und
- Figur 9: - die schematische Ansicht einer weiteren Knieorthese.

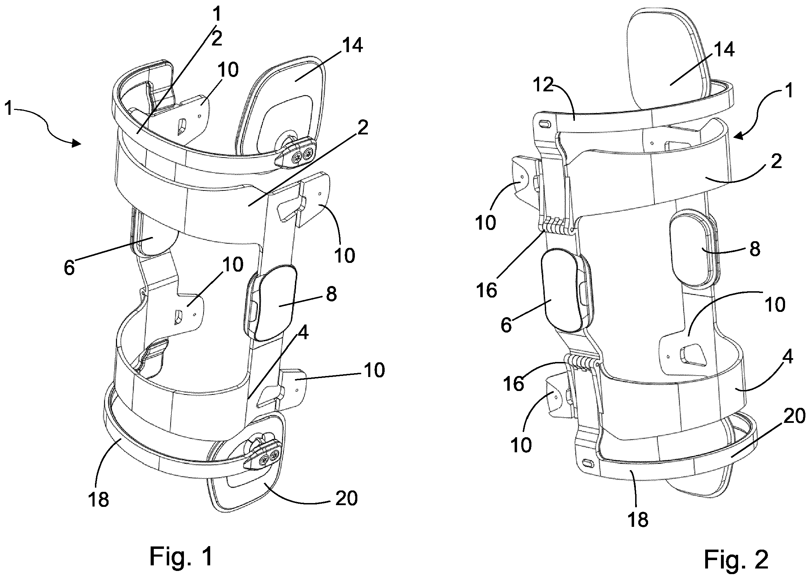

Die Figuren 1 und 2 zeigen eine Knieorthese 1 gemäß einem Ausführungsbeispiel der vorliegenden Erfindung. Sie verfügen über einen proximalen Rahmen 2 sowie einen distalen Rahmen 4. Der proximale Rahmen 2 und der distale Rahmen 4 sind durch ein Lateralgelenk 6 und ein Medialgelenk 8 miteinander schwenkbar verbunden. Das Lateralgelenk 6 und das Medialgelenk 8 erlauben jeweils eine Bewegung entlang einer Sagittalebene. Die Flexion und Extension eines Knies wird auf diese Weise nicht behindert.

Sowohl am proximalen Rahmen 2 als auch am distalen Rahmen 4 befinden sich Befestigungslaschen 10, an denen ein Gurt befestigt werden kann, um die Knieorthese 1 am Bein des Trägers zu befestigen.

Die Knieorthese 1 verfügt zudem über einen proximalen Stützarm 12, an dem sich ein proximales Druckelement 14 befindet. Der proximale Stützarm 12 ist lateral über eine Einstelleinrichtung 16 an dem proximalen Rahmen 2 angeordnet.

Am distalen Rahmen 4 ist über eine zweite Einstelleinrichtung 16 ein distaler Stütz-arm 18 angeordnet, an dem sich ein distales Druckelement 20 befindet.

Bei dem proximalen Druckelement 14 und dem distalen Druckelement 20 handelt es sich um ein Kissen oder eine Pelotte, die am Bein des Trägers anliegt.

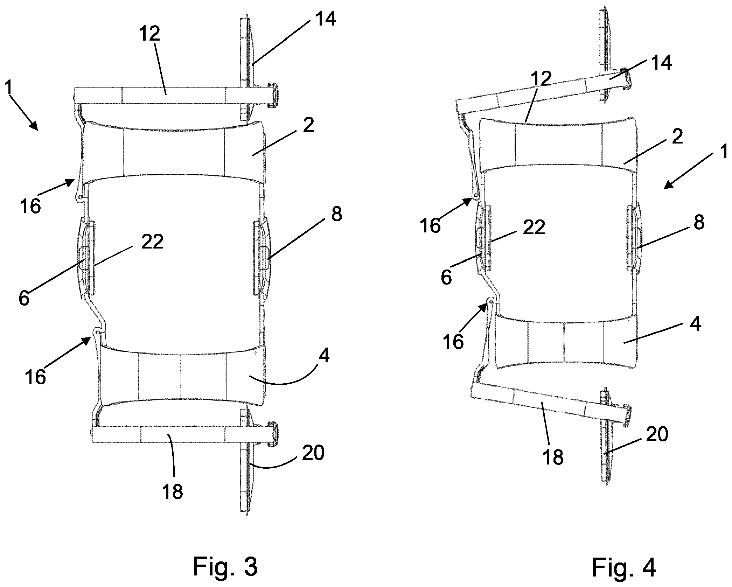

Die Figuren 3 und 4 zeigen eine Frontalansicht der Knieorthese 1 aus den Figuren 1 und 2. Der proximale Rahmen 2 ist mit dem distalen Rahmen 4 über das Lateralgelenk 6 und das Medialgelenk 8 verbunden. Die Figuren 3 und 4 zeigen zwei unterschiedliche Winkeleinstellungen der Einstelleinrichtungen 16. Während der proximale Stützarm 12 und der distale Stützarm 18 in Figur 3 horizontal verlaufen, sind sie in Figur 4 relativ stark geneigt. Dies hat zur Folge, dass das proximale Druckelement 14 und das distale Druckelement 20 in Figur 4 im Vergleich zu Figur 3 nach lateral, in der gezeigten Ansicht also nach links, verschoben sind. Auf ein Bein, das sich in der Knieorthese 1 befindet, wird in Figur 4 eine deutlich stärkere Kraft in Lateralrichtung ausgeübt, als dies in Figur 3 der Fall ist. Selbstverständlich ist es auch möglich, ein proximalen Stützarm 12 und einen distalen Stützarm 18 zu wählen, die so ausgebildet sind, dass sich das proximale Druckelement 14 und das distale Druckelement 20 auf der Medialseite befinden. Ein drittes Druckelement 22 befindet sich im gezeigten Ausführungsbeispiel auf der dem Knie zugewandten Seite des Lateralgelenkes 6.

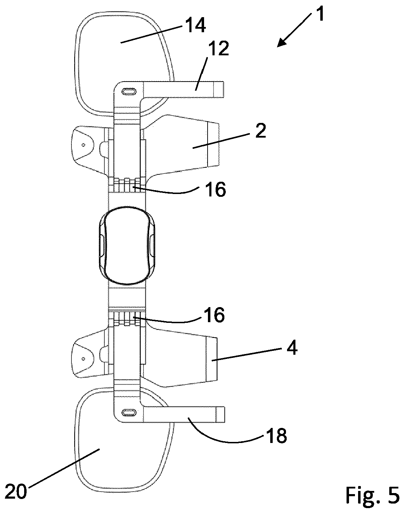

Figur 5 zeigt eine Lateralseitenansicht der Knieorthese 1. Man erkennt die Einstelleinrichtungen 16, über die der proximale Stützarm 12 mit dem sich daran befinden-den proximalen Druckelement 14 sowie der distale Stützarm 18 mit dem sich daran befindenden distalen Druckelement 20 an dem jeweiligen proximalen Rahmen 2 und dem distalen Rahmen 4 angeordnet ist.

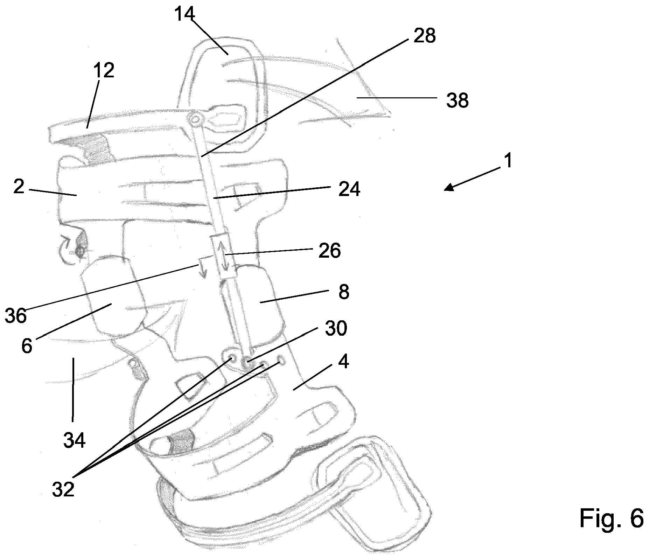

Figur 6 zeigt die Knieorthese 1, bei der anders als in den bisher gezeigten Knieorthesen zwischen dem distalen Rahmen 4 und dem proximalen Stützarm 12 ein Koppelelement 24 angeordnet ist. Das Koppelelement 24 ist im gezeigten Ausführungsbeispiel eine längenveränderbare Stange, die über eine Längenveränderungseinrichtung 26 verfügt. Durch diese Längenveränderungseinrichtung 26 ist es möglich, die Länge des Koppelelementes 24 zu verändern und das gewünschte Maß einzustellen und nach dem Einstellen die Länge wieder zu fixieren, sodass die Hauptaufgabe des Koppelelementes, nämlich Zug- oder Druckkräfte zu übertragen, erfüllt werden kann.

Ein erstes Ende 28 des Koppelelementes 24 ist am proximalen Stützarm 12 angeordnet. Ein dem ersten Ende 28 gegenüberliegendes zweites Ende 30 ist am distalen Rahmen 4 positioniert. Dies kann in unterschiedlichen Positionen geschehen. Im gezeigten Ausführungsbeispiel verfügt der distale Rahmen 4 dazu über vier Löcher 32, in denen das zweite Ende 30 des Koppelelementes 24 befestigt werden kann.

In Figur 6 ist der Effekt des Koppelelementes 24 bei einer Flexion, also dem Einbeugen des Knies, gezeigt. Dies wird durch den ersten Pfeil 34 dargestellt. Diese Schwenkbewegung des distalen Rahmens 4 relativ zum proximalen Rahmen 2 hat zur Folge, dass das zweite Ende 30 und damit das Koppelelement 24 in Figur 6 nach unten bewegt wird, was durch den zweiten Pfeil 36 dargestellt wird. Diese Zugkraft, die durch das Koppelelement 24 auf den proximalen Stützarm 12 übertragen wird, führt dazu, dass das proximale Druckelement 14 vom Bein des Trägers wegbewegt wird oder dass sich zumindest die durch das proximale Druckelement 14 aufgebrachte Kraft verringert. Dies wird durch den dritten Pfeil 38 dargestellt.

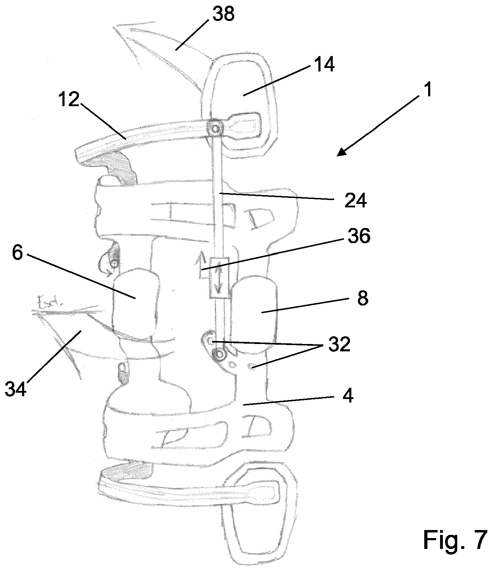

Figur 7 zeigt die Orthese aus Figur 6 bei der Extensionsbewegung. Auch hier ist das Koppelelement 24 am proximalen Stützarm 12 und am distalen Rahmen 4 angeordnet. Die Extension wird durch den ersten Pfeil 34 angedeutet. Durch diese Schwenkbewegung wird dem zweiten Pfeil 36 entsprechend das Koppelelement 24 nach oben bewegt, wodurch eine Druckkraft auf den proximalen Stützarm übertragen wird. Dieser hat zur Folge, dass das proximale Druckelement 14 in Richtung auf das Bein bewegt wird oder dass sich zumindest die aufgebrachte Kraft erhöht. Dies wird durch den dritten Pfeil 38 dargestellt.

Der Kraftverlauf lässt sich durch die Wahl eines Anlenkpunktes des ersten Endes 28 und/oder des zweiten Endes 30 des Koppelelementes 24 beeinflussen. Wird das zweite Ende 30 des Koppelelementes 24 beispielsweise in das rechte der Löcher 32 eingesteckt oder an diesen befestigt, verläuft das Koppelelement 24 nicht wie in den Figuren 6 und 7 gezeigt vor der Schwenkachse des Lateralgelenkes 6 und des Medialgelenkes 8, sondern hinter der entsprechenden Schwenkachse. Dadurch kann beispielsweise erreicht werden, dass bei einer Flexion des Gelenkes, wie sie in Figur 6 dargestellt wird, nicht eine Zugkraft durch das Koppelelement 24 auf den proximal Stützarm 12 übertragen wird, sondern eine Druckkraft. Durch geschickte Wahl eines Anlenkpunktes, beispielsweise der Position eines der Löcher 32, lässt sich der Kraftverlauf nahezu beliebig einstellen. Insbesondere lässt sich erreichen, dass die durch das proximale Druckelement 14 aufgebrachte Kraft beispielsweise bei einer Flexion des Kniegelenkes zunächst ansteigt und bei

Überschreiten eines maximal Schwenkwinkels, bei dem die Kraft maximal wird, wieder abfällt.

Das Gesagte gilt analog für eine Knieorthese 1, bei der das Koppelelement 24 am proximalen Rahmen 2 und dem distalen Stützarm 18 befestigt ist. In diesem Fall wird beim Beugen oder Strecken des Knies die durch das distale Druckelement 20 aufgebrachte Kraft variiert.

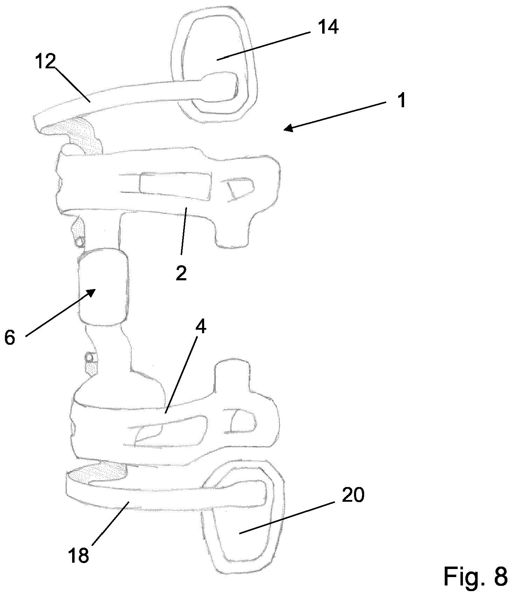

Figur 8 zeigt eine Knieorthese 1, die lediglich ein Lateralgelenk 6 und kein Medialgelenk 8 aufweist. Dennoch erstreckt sich der proximale Rahmen 2 sowie der distale Rahmen 4 von der medialen Seite bis zur lateralen Seite der Knieorthese 1 und im angelegten Zustand, der in Figur 8 nicht dargestellt ist, auch von der medialen Seite bis zur lateralen Seite des Beines und damit des Knies. Der proximale Stützarm 12, an dem sich das proximale Druckelement 14 befindet, ist auf der Lateralseite am proximalen Rahmen 2 angeordnet. Analog dazu ist der distale Stützarm 18, an dem sich das distale Sützelement 20 befindet, ebenfalls auf der lateralen Seite des distalen Rahmens 4 angeordnet.

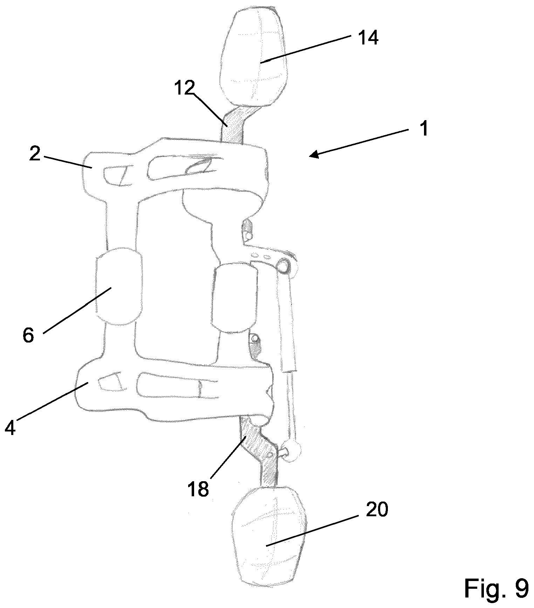

Figur 9 zeigt eine weitere Ausführungsform einer Knieorthese 1, bei der der proximale Stützarm 12 und der distale Stützarm 18 auf der gleichen Seite, beispielsweise lateral, an dem proximalen Rahmen 2 oder dem distalen Rahmen 4 angeordnet sind. Das proximale Druckelement 14 und das distale Druckelement 20 befinden sich auf der gleichen Seite, im gezeigten Beispiel also ebenfalls lateral.

### Bezugszeichenliste

- 1: Knieorthese
- 2: proximaler Rahmen
- 4: distaler Rahmen
- 6: Lateralgelenk
- 8: Medialgelenk
- 10: Befestigungslasche

- 12: proximaler Stützarm
- 14: proximales Druckelement
- 16: Einstelleinrichtung
- 18: distaler Stützarm
- 20: distales Druckelement

- 22: drittes Druckelement
- 24: Koppelelement
- 26: Längenveränderungseinrichtung
- 28: erstes Ende
- 30: zweites Ende

- 32: Loch
- 34: erster Pfeil
- 36: zweiter Pfeil
- 38: dritter Pfeil

## Patentansprüche

1. Knieorthese (1) mit
a. einem proximalen Rahmen (2) und
b. einem distalen Rahmen (4),
die durch ein Lateralgelenk (6) und/oder ein Medialgelenk (8) schwenkbar aneinander angeordnet sind,
wobei
die Knieorthese (1) ein proximales Druckelement (14), das mittels eines proximalen Stützarmes (12) ausschließlich lateral oder ausschließlich medial an dem proximalen Rahmen (2) angeordnet ist, und
ein distales Druckelement (20) aufweist, das mittels eines distalen Stützarmes (18) ausschließlich lateral oder ausschließlich medial an dem distalen Rahmen (4) angeordnet ist,
**dadurch gekennzeichnet, dass** die Knieorthese (1) ein Koppelelement (24) aufweist, das mit einem ersten Ende an dem proximalen Stützarm (12) und mit einem zweiten Ende an dem distalen Rahmen (4) oder mit dem ersten Ende an dem distalen Stützarm (18) und mit dem zweiten Ende an dem proximalen Rahmen (2) angeordnet ist.

2. Knieorthese (1) nach Anspruch 1, **dadurch gekennzeichnet, dass** der proximale Stützarm (12) und der distale Stützarm (18) auf der gleichen Seite an dem jeweiligen Rahmen (2, 4) angeordnet ist.

3. Knieorthese (1) nach Anspruch 1 oder 2, **dadurch gekennzeichnet, dass** ein Winkel, in dem der proximale Stützarm (12) und/oder der distale Stützarm (18) an dem jeweiligen Rahmen (2, 4) angeordnet ist, über jeweils eine Einstelleinrichtung (16) einstellbar ist.

4. Knieorthese (1) nach Anspruch 3, **dadurch gekennzeichnet, dass** der Winkel für den proximalen Stützarm (12) und für den distalen Stützarm (18) unabhängig voneinander einstellbar ist.

5. Knieorthese (1) nach Anspruch 3 oder 4, **dadurch gekennzeichnet, dass** die Einstelleinrichtungen (16) arretierbar sind.

6. Knieorthese (1) nach einem der vorstehenden Ansprüche, **dadurch gekennzeichnet, dass** das Lateralgelenk (6) und das Medialgelenk (8) eine Schwenkbewegung des proximalen Rahmens (2) relativ zu dem distalen Rahmens (4) ausschließlich in einer Ebene erlauben, die im angelegten Zustand der Knieorthese (1) im Wesentlichen einer Sagittalebene entspricht.

7. Knieorthese (1) nach einem der vorstehenden Ansprüche, **dadurch gekennzeichnet, dass** der proximale Stützarm (12) und/oder der distale Stützarm (18) lösbar an dem jeweiligen Rahmen (2, 4) angeordnet ist.

8. Knieorthese (1) nach einem der vorstehenden Ansprüche, **dadurch gekennzeichnet, dass** das proximale Druckelement (14) und das distale Druckelement (20) derart angeordnet sind, dass sie im angelegten Zustand der Knieorthese (1) beide medial oder beide lateral an einem Bein des Trägers anliegen.

9. Knieorthese (1) nach einem der vorstehenden Ansprüche, **dadurch gekennzeichnet, dass** die Druckelemente (14, 20) schwenkbar an den Stützarmen (12, 18) angeordnet sind.

10. Knieorthese (1) nach einem der vorstehenden Ansprüche, **dadurch gekennzeichnet, dass** die Druckelemente (14, 20) lösbar an den Stützarmen (12, 18) angeordnet sind.

11. Knieorthese (1) nach einem der vorstehenden Ansprüche, **dadurch gekennzeichnet, dass** das Koppelelement derart angeordnet ist, dass eine im angelegten Zustand der Knieorthese (1) von dem proximalen Druckelement (14) auf den Oberschenkel oder von dem distalen Druckelement (20) auf den Unterschenkel aufgebrachte Kraft durch das Koppelelement beim Beugen des Knies variiert.

12. Knieorthese (1) nach Anspruch 11, **dadurch gekennzeichnet, dass** die Kraft bei einem Schwenkwinkel maximal ist, der größer als 10°, bevorzugt größer als 15°, besonders bevorzugt größer als 17° und kleiner als 30°, bevorzugt kleiner als 25°, besonders bevorzugt kleiner als 22° ist.

13. Knieorthese (1) nach Anspruch 12, **dadurch gekennzeichnet, dass** der Schwenkwinkel 20° ist.

14. Knieorthese (1) nach einem der vorstehenden Ansprüche, **dadurch gekennzeichnet, dass** eine Länge des Koppelelemente veränderbar und feststellbar ist.

15. Knieorthese (1) nach einem der vorstehenden Ansprüche, **dadurch gekennzeichnet, dass** sich das proximale Druckelement (14) auf der Seite befindet, auf der der proximale Stützarm (12) am proximalen Rahmen (2) angeordnet ist und/oder dass sich das distale Druckelement (20) auf der Seite befindet, auf der der distale Stützarm (18) am proximalen Rahmen (4) angeordnet ist.

## Claims

1. A knee orthosis (1) with
a. a proximal frame (2) and
b. a distal frame (4),
which are connected via a lateral joint (6) and/or a medial joint (8) such that they can be swivelled, wherein
the knee orthosis (1) comprises a proximal pressure element (14), which is arranged exclusively laterally or exclusively medially on the proximal frame (2) by means of a proximal support arm (12), and
a distal pressure element (20), which is arranged exclusively laterally or exclusively medially on the distal frame (4) by means of a distal support arm (18), **characterized in that** the knee orthosis (1) features a coupling element (24) which is arranged with a first end on the proximal support arm (12) and with a second end on the distal frame (4), or with the first end on the distal support arm (18) and with the second end on the proximal frame (2).

2. The knee orthosis (1) according to claim 1, **characterized in that** the proximal support arm (12) and the distal support arm (18) are arranged on the same side on the respective frame (2, 4).

3. The knee orthosis (1) according to claim 1 or 2, **characterized in that** an angle at which the proximal support arm (12) and/or the distal support arm (18) is arranged on the respective frame (2, 4) can each be adjusted using an adjustment device (16).

4. The knee orthosis (1) according to claim 3, **characterized in that** the angle for the proximal support arm (12) and for the distal support arm (18) can be adjusted independently of each other.

5. The knee orthosis (1) according to claim 3 or 4, **characterized in that** the adjustment devices (16) can be locked.

6. The knee orthosis (1) according to one of the preceding claims, **characterized in that** the lateral joint (6) and the medial joint (8) allow a swivel movement of the proximal frame (2) relative to the distal frame (4) exclusively in a plane which essentially corresponds to a sagittal plane when the knee orthosis (1) is in the mounted state.

7. The knee orthosis (1) according to one of the preceding claims, **characterized in that** the proximal support arm (12) and/or the distal support arm (18) are arranged on the respective frame (2, 4) such that they can be detached.

8. The knee orthosis (1) according to one of the preceding claims, **characterized in that** the proximal pressure element (14) and the distal pressure element (20) are arranged in such a way that they both lie medially or they both lie laterally on a leg of the wearer when the knee orthosis (1) is in the mounted state.

9. The knee orthosis (1) according to one of the preceding claims, **characterized in that** the pressure elements (14, 20) are arranged on the support arms (12, 18) such that they can be swivelled.

10. The knee orthosis (1) according to one of the preceding claims, **characterized in that** the pressure elements (14, 20) are arranged on the support arms (12, 18) such that they can be detached.

11. The knee orthosis (1) according to one of the preceding claims, **characterized in that** the coupling element is arranged in such a way that a force applied by the proximal pressure element (14) to the upper leg or by the distal pressure element (20) to the lower leg when the knee orthosis (1) is in the mounted state is varied by the coupling element when the knee is bent.

12. The knee orthosis (1) according to claim 11, **characterized in that** the force reaches its maximum at a swivel angle that is greater than 10°, preferably greater than 15°, particularly preferably greater than 17° and less than 30°, preferably less than 25°, particularly preferably less than 22°.

13. The knee orthosis (1) according to claim 12, **characterized in that** the swivel angle is 20°.

14. The knee orthosis (1) according to one of the preceding claims, **characterized in that** a length of the coupling element can be changed and fixed.

15. The knee orthosis (1) according to one of the above claims, **characterized in that** the proximal pressure element (14) is located on the side on which the proximal support arm (12) is arranged on the proximal frame (2) and/or that the distal element (20) is located on the side on which the distal support arm (18) is arranged on the proximal frame (4).

## Revendications

1. Orthèse de genou (1) comprenant
a. un cadre proximal (2) et
b. un cadre distal (4),
qui sont disposés l'un sur l'autre de manière à pouvoir pivoter par l'intermédiaire d'une articulation latérale (6) et/ou d'une articulation médiale (8),
dans laquelle
l'orthèse de genou (1) comprend
un élément de compression proximal (14) qui est disposé exclusivement latéralement ou exclusivement médialement sur le cadre proximal (2) au moyen d'un bras de soutien proximal (12), et
un élément de compression distal (20) qui est disposé exclusivement latéralement ou exclusivement médialement sur le cadre distal (4) au moyen d'un bras de soutien distal (18),
**caractérisée en ce que** l'orthèse de genou (1) présente un élément de couplage (24) qui est disposé avec une première extrémité sur le bras de soutien proximal (12) et avec une deuxième extrémité sur le cadre distal (4) ou avec la première extrémité sur le bras de soutien distal (18) et avec la deuxième extrémité sur le cadre proximal (2).

2. Orthèse de genou (1) selon la revendication 1,
**caractérisée en ce que** le bras de soutien proximal (12) et le bras de soutien distal (18) sont disposés du même côté sur le cadre respectif (2, 4).

3. Orthèse de genou (1) selon la revendication 1 ou 2,
**caractérisée en ce qu'**un angle selon lequel le bras de soutien proximal (12) et/ou le bras de soutien distal (18) est disposé sur le cadre respectif (2, 4) est réglable par l'intermédiaire d'un dispositif de réglage respectif (16).

4. Orthèse de genou (1) selon la revendication 3,
**caractérisée en ce que** ledit angle est réglable pour le bras de soutien proximal (12) et pour le bras de soutien distal (18) indépendamment l'un de l'autre.

5. Orthèse de genou (1) selon la revendication 3 ou 4,
**caractérisée en ce que** les dispositifs de réglage (16) peuvent être bloqués.

6. Orthèse de genou (1) selon l'une des revendications précédentes, **caractérisée en ce que** l'articulation latérale (6) et l'articulation médiale (8) permettent un mouvement de pivotement du cadre proximal (2) par rapport au cadre distal (4) exclusivement dans un plan qui, dans l'état appliqué de l'orthèse de genou (1), correspond sensiblement à un plan sagittal.

7. Orthèse de genou (1) selon l'une des revendications précédentes, **caractérisée en ce que** le bras de soutien proximal (12) et/ou le bras de soutien distal (18) est disposé de manière amovible sur le cadre respectif (2, 4).

8. Orthèse de genou (1) selon l'une des revendications précédentes, **caractérisée en ce que** l'élément de compression proximal (14) et l'élément de compression distal (20) sont disposés de telle sorte que, dans l'état appliqué de l'orthèse de genou (1), ils s'appuient tous les deux médialement ou tous les deux latéralement sur une jambe du porteur.

9. Orthèse de genou (1) selon l'une des revendications précédentes, **caractérisée en ce que** les éléments de compression (14, 20) sont disposés sur les bras de soutien (12, 18) de manière à pouvoir pivoter.

10. Orthèse de genou (1) selon l'une des revendications précédentes, **caractérisée en ce que** les éléments de compression (14, 20) sont disposés de manière amovible sur les bras de soutien (12, 18).

11. Orthèse de genou (1) selon l'une des revendications précédentes, **caractérisée en ce que** l'élément de couplage est disposé de telle sorte qu'une force appliquée par l'élément de compression proximal (14) sur la cuisse ou par l'élément de compression distal (20) sur le bas de jambe, dans l'état appliqué de l'orthèse de genou (1), varie par l'intermédiaire de l'élément de couplage lors de la flexion du genou.

12. Orthèse de genou (1) selon la revendication 11,
**caractérisée en ce que** la force est maximale à un angle de pivotement supérieur à 10°, de préférence supérieur à 15°, de manière particulièrement préférée supérieur à 17° et inférieur à 30°, de préférence inférieur à 25°, de manière particulièrement préférée inférieur à 22°.

13. Orthèse de genou (1) selon la revendication 12,
**caractérisée en ce que** l'angle de pivotement est de 20°.

14. Orthèse de genou (1) selon l'une des revendications précédentes, **caractérisée en ce qu'**une longueur de l'élément de couplage peut être modifiée et fixée.

15. Orthèse de genou (1) selon l'une des revendications précédentes, **caractérisée en ce que** l'élément de compression proximal (14) est situé du côté où le bras de soutien proximal (12) est disposé sur le cadre proximal (2), et/ou **en ce que** l'élément de compression distal (20) est situé du côté où le bras de soutien distal (18) est disposé sur le cadre proximal (4).
